# EUROPEAN PATENT SPECIFICATION

(11) **EP 1 384 998 B1**
(45) Date of publication and mention of the grant of the patent: **11.10.2006**
(21) Application number: 02713281.0
(22) Date of filing: 01.04.2002
(51) Int. Cl.: G01N 33/50, G01N 33/15

(54) **EYESTRAIN MODEL, METHOD OF CONSTRUCTING THE SAME, EVALUATION METHOD WITH THE USE OF THE MODEL AND DRUGS SCREENED BY USING THE EVALUATION METHOD**
MODELL FÜR AUGEN-ÜBERANSTRENGUNG, VERFAHREN ZU DESSEN KONSTRUKTION, AUSWERTUNGSVERFAHREN UNTER VERWENDUNG DES MODELLS SOWIE ARZNEIMITTEL, DIE MIT DIESEN AUSWERTUNGSVERFAHREN GESCREENT WURDEN
MODELE DE FATIGUE OCULAIRE, PROCEDE DE REALISATION ASSOCIE, PROCEDE D'EVALUATION A L'AIDE DU MODELE ET MEDICAMENTS CRIBLES A L'AIDE DU PROCEDE D'EVALUATION

(30) Priority: 26.04.2001 JP 2001130414; 26.02.2002 JP 2002050116
(43) Date of publication of application: 28.01.2004
(73) Proprietor: Biochemical and Pharmacological Laboratories Inc., Tondabayashi-shi, Osaka 584-0023 (JP)
(72) Inventor: KATSUYAMA, Iwao, Biochemical & Pharma. Lab. Inc., Tondabayashi-shi, Osaka 584-0023 (JP)
(74) Representative: Schüssler, Andrea
(86) International application number: PCT/JP2002/003277
(87) International publication number: WO 2002/090980

(56) References cited:
- EP-A- 0 819 431
- EP-A- 1 034 793
- JP-A- 7 133 225
- PATENT ABSTRACTS OF JAPAN vol. 1997, no. 10, 31 October 1997 (1997-10-31) & JP 09 143099 A (SANTEN PHARMACEUT CO LTD), 3 June 1997 (1997-06-03)
- PATENT ABSTRACTS OF JAPAN vol. 1997, no. 07, 31 July 1997 (1997-07-31) & JP 09 059173 A (SANTEN PHARMACEUT CO LTD), 4 March 1997 (1997-03-04)

## Description

### TECHNICAL FIELD

The present invention relates to a model for asthenopia, a method of generating the model, an evaluation method using the model, a medicine selected using the evaluation method. More specifically, the present invention relates to a model which is used in the evaluation of a medicine of which therapeutic purpose is amelioration of fine accommodations in accommodative asthenopia, a method of generating the model, an evaluation method using the model, a medicine selected by using the evaluation method.

### BACKGROUND ART

Asthenopia is caused when VDT (Visual Display Terminal) operations are carried out for a long period of time, when focusing is frequently changed in a long-distance driving, or the like.

This asthenopia is said to be caused by difficulty in smooth accommodation when the ciliary muscle is fatigued due to over-tension (*Journal of Japanese Ophthalmology Association (Nihon Gankagakukai Zasshi)* **92**, 1854-1858 (1988)). The ciliary muscle as referred to herein is a smooth muscle which makes up a major constituent of the ciliary muscles surrounding the crystalline lens in a ring-shaped form in ocular tissues.

In the case of the ciliary muscle, the contraction by the stimulation against the ciliary muscle is a simple contraction, and its contraction form is known to be isometric, in which the muscle is contracted while both ends of the muscle are fixed to a given length. And if the muscle is continued to be subjected to repeated stimulations, the tension of the muscle contraction is gradually declined, and at the same time the contraction process is delayed. The phenomenon is referred to as muscle fatiguing, and asthenopia is caused by fatigue of the ciliary muscle.

Medicines such as eye drop have been used to treat asthenopia.

However, a medicine which can surely treat asthenopia has not existed so far. Also, a method for evaluating the medicine has not yet been established.

Currently, there are various methods for evaluating a medicine. Tests to be conducted prior to clinical test are roughly classified into *in vivo* system and *in vitro* system. Especially the *in vitro* tests are widely used because of simplicity and rapidness at an initial stage of the evaluation.

In the case of the determination by *in vitro* system, the fatigue of ciliary muscle is shown by the phenomena of decline in contraction of muscles and delay in contraction/relaxation process. Therefore, the fatigue can be determined by direct observation.

Concretely, "Magnus" method which is subjected to an enucleated ciliary muscle may be used.

In the Magnus method, there is usually used a Magnus apparatus comprising as main constituents: a Magnus tube which is a thin tube of about 1 to 2 s in diameter and about 2 to 5 cm in depth for immersion of an organ in an artificial nutrient solution; an air inlet tube for introducing the air to the artificial nutrient solution; an anchor portion for fixing one end of the organ; a tension transducer for fixing another end of the organ and measuring the tension of the organ; and a thermostat for keeping the temperature of the Magnus tube at a given temperature (37°C in most cases).

The Magnus method is advantageous for carrying out measurement under physiological conditions, because the enucleated organ is free from the influences of other organs of a living body, like the case where an organism in such state is subjected to a test, and yet does not lose its mobility owing to the nerve plexus remaining in the muscle even when the organ is removed from the control of the central nerves.

As those studying pharmacological actions by the Magnus method, there have been reported a case where M₃-type muscarinic receptor in the bovine ciliary muscle is studied (Hiroshi Masuda et al., *Gen. Pharmac*., **30(4)**, 579-584 (1998)), a case where a relaxation response of the muscle to nitrous oxide in the bovine ciliary muscle is studied (Soichiro Kamikawa et al., *Exp. Eye Res*., **66**, 1-7 (1998)), (Hiroshi Masuda et al., *Current Eye Research*, **16, (12),** 1245-1251 (1997)) and the like.

Furthermore, it is disclosed in Japanese Patent Laid-Open No. Hei 7-133225 a case where ciliary muscle tension-relaxation agent is evaluated using a ciliary muscle sample in which the muscle is transiently contracted with potassium chloride or carbachol.

However, the ciliary muscle sample obtained by the above is ciliary muscle in a contraction state with a contraction-inducing substance, which could be hardly said to be ciliary muscle with the true fatiguing.

Therefore, in the evaluation of a medicine on a sample in which fatigue could not be reproduced as in a living body, a justified evaluation cannot be said to be made.

Furthermore, in the above disclosure, since only the evaluation for an antagonist against the contraction-inducing substance can be made, its effect could not be relatively evaluated with other medicines for ameliorating asthenopia.

Therefore, in view of the above problems, as a result of conducting intensive studies, the present inventors have found a sample in which the ciliary muscle in fatiguing occurring in a living body is reproduced in *in vitro* system and a method of generating the sample. Also, the present inventors have found a method for directly and objectively evaluating a pharmacological effect of a medicine for ameliorating asthenopia, and a method of relatively comparing with other medicines. The present invention has been perfected thereby.

### DISCLOSURE OF INVENTION

An invention according to claim 1 relates to a model for asthenopia, wherein a muscular contraction of ciliary muscle is substantially stably decreased by contraction response with plural stimulations.

An invention according to claim 2 relates to the model according to claim 1, wherein the asthenopia is accommodative asthenopia.

An invention according to claim 3 relates to the model according to claim 1 or 2, wherein a ciliary muscle sample enucleated from a non-human mammal or fowl is used.

An invention according to claim 4 relates to the model according to claim 1 or 2, wherein a ciliary muscle sample enucleated from a non-human mammal is used.

An invention according to claim 5 relates to the model according to any one of claims 1 to 4, wherein the ciliary muscle is contracted a plurality of times by the use of smooth muscle contraction-inducing means.

An invention according to claim 6 relates to the model according to any one of claims 1 to 5, wherein the smooth muscle contraction-inducing means is chemical stimulus with a chemical substance.

An invention according to claim 7 relates to the model according to claim 6, wherein the chemical substance is selected from acetylcholine, serotonin, histamine, muscarine and endothelin.

An invention according to claim 8 relates to the model according to any one of claims 1 to 5, wherein the smooth muscle contraction-inducing means is electrical stimulus.

An invention according to claim 9 relates to the model according to any one of claims 1 to 8, wherein the contraction response with stimulations is repeated at least three times, thereby giving a substantially stable decrease of muscular contraction.

An invention according to claim 10 relates to the model according to any one of claims 1 to 9, wherein the ciliary muscle shows a decrease ratio of muscular contraction of 50 ± 30%.

An invention according to claim 11 relates to the model according to any one of claims 1 to 9, wherein the ciliary muscle shows a decrease ratio of muscular contraction of 50 ± 20%.

An invention according to claim 12 relates to the model according to any one of claims 1 to 9, wherein the ciliary muscle shows a decrease ratio of muscular contraction of 50 ± 10%.

An invention according to claim 13 relates to a method of generating the model for asthenopia as defined in any one of claims 1 to 12, wherein a muscular contraction of ciliary muscle is substantially stably decreased by contraction response with plural stimulations.

An invention according to claim 14 relates to a method for evaluating a medicine against asthenopia, characterized by contacting in the model for asthenopia as defined in any one of claims 1 to 12 with a medicine, and measuring a contraction ratio of ciliary muscle, thereby evaluating an effect of ameliorating asthenopia by the medicine.

An invention according to claim 15 relates to the method according to claim 14, characterized by measuring contraction ratios of ciliary muscle before and after administration of the medicine, and comparing the contraction ratios.

An invention according to claim 16 relates to the method according to claim 14 or 15, characterized by carrying out the method by the use of a Magnus apparatus.

An invention according to claim 17 relates to a medicine selected using the method as defined in any one of claims 14 to 16.

### BRIEF DESCRIPTION OF THE DRAWINGS

Figure 1 is a graph showing a change in the contraction ratio by acetylcholine stimulation in the generation of samples and in the preliminary test.
Figure 2 is a graph showing the comparison of the contraction ratio by a tenth acetylcholine stimulation in the preliminary test.
Figure 3 a graph showing a change in the contraction ratio by acetylcholine stimulation in the main test.
Figure 4 is a graph showing the comparison of the contraction ratio by a tenth acetylcholine stimulation in the main test.

### BEST MODE FOR CARRYING OUT THE INVENTION

The medicine for ameliorating asthenopia to be evaluated in the present invention is usually a drug or quasi-drug, and there may be included designated health-care foods (functional foods), food supplements such as health-care foods, foods and the like.

The generation of the sample and the evaluation method therefor will be explained hereinbelow.

First, a specimen to be tested, which is a sample, is prepared.

A method for obtaining ciliary muscle which is the specimen to be tested is not particularly limited, and includes, for instance, the following method.

An eyeball is enucleated from a test animal under general anesthesia. The enucleated eyeball is sclerotomized and amputated into half at the equatorial position. Thereafter, the ciliary muscle is carefully ablated from sclera after removal of the crystalline lens, to give ciliary muscle.

As the test animal from which the ciliary muscle is obtained, there can be used non-human mammals or fowls, which can be exemplified by *Bos*, sheep, pigs, goats, monkeys, dogs, cats, rabbits, guinea pigs, rats, mice, *Gallus*, geese, quails, ostriches and the like. Among them, one of non-human mammals is preferably used.

It is preferable that the test animals before the enucleation of the ciliary muscle are bred under the same conditions according to a conventional method to obtain samples of the ciliary muscle in as much the same state as possible.

After the enucleation of the ciliary muscle, the ciliary muscle obtained may be cut to a desired size to generate a sample. The size of the sample is not particularly limited. For example, if the sample is to be reacted in a Magnus tube described later, a cut piece of about 3 mm in width and about 6 mm in length is preferable.

Secondly, stimuli are given a plurality of times to the enucleated ciliary muscle, to induce a contraction response to the ciliary muscle. The stimuli used are not particularly limited as long as the contraction response to the ciliary muscle is induced. Since the ciliary muscle is a kind of smooth muscle, it is preferable to use a smooth muscle contraction-inducing means. The means of inducing smooth muscle contraction as referred to in the present invention is stimulus for inducing reversible contraction in a smooth muscle. Examples thereof include physical stimuli, chemical stimuli and electrical stimuli. Among them, it is preferable to use the chemical stimuli or the electric stimuli which control contraction/relaxation of the ciliary muscle in a living body.

Examples of substances which can be used for the chemical stimuli include acetylcholine, serotonin, histamine, muscarine, nicotine and endothelin. Among them, acetylcholine is preferably used.

It has been known that acetylcholine is released from the parasympathetic nerve and stimulates a cholinergic receptor to induce strong contraction in the smooth muscle.

Serotonin is a promising candidate for an intracerebral chemical transmitter, which exists in the intestinal chromaffin cell of the intestinal mucosa and migrates into a blood platelet from the chromaffin cell. Especially, there has been known to induce contraction response in vascular smooth muscle in the pulmonary circulation or in smooth muscles such as enucleated intestinal canal and muscularis of the bronchus.

Histamine is stored in a basophil in the blood and in a tissue mast cell, which plays a key role in inflammation and allergic response. There may be caused the accentuation of intestinal peristalsis, the acceleration of gastric acid secretion, and the accentuation of dilation and permeability of minute capillary blood vessels. Especially, there has been known to show a strong contraction of bronchus and to have contraction action of smooth muscle, e.g., in blood vessels.

Muscarine is an alkaloid of the toadstool origin and like acetylcholine, stimulates a cholinergic receptor, of which action is blocked by atropine. In other words, there has been known to act on postsynaptic membranes to induce the strong contraction of bronchus and to have contraction action of smooth muscle, e.g., in blood vessels.

Nicotine is an alkaloid obtained from tobacco leaves, and like acetylcholine, stimulates a cholinergic receptor, of which action is blocked by hexamethonium. In other words, there has been known to act on the neuromuscular synapses of autonomic ganglia and motor end plates, and to induce a strong contraction of bronchus and to have contraction action of smooth muscle, e.g., in blood vessels.

Endothelin is a polypeptide consisting of 21 amino acids of which production in human or pig epithelial cell has been confirmed. There has been known to have the strong contraction action of blood vessels and a long-lasting potent hypertensive action (*Nature*, **332**, 411-415 (1988); *FEBS Letters,* **231**, 440-444 (1988)).

The concentration of the chemical substance used in the chemical stimuli may be appropriately set depending upon the kinds of chemical substances used or their purposes or situations. Among them, the concentration is preferably from 10⁻¹⁰ to 10⁻¹ mol/L, more preferably from 10⁻⁹ to 10⁻² mol/L.

When the electrical stimuli are used for generating the model of the present invention, the intensity of the stimuli can be changed by appropriately setting various conditions such as the method of electric loading (direct stimulation or indirect stimulation), kinds of electric current (alternate current, direct current or the like), or the intensity of the electric current or voltage, the processing period of time, the number of processing and the intervals between each processing. The conditions used may be appropriately adjusted depending upon the degree of fatigue to be induced in the sample, and are not particularly limited.

When the electric stimuli are used, the degree of electric stimulation may fluctuate depending on various factors such as an apparatus used, the kinds of electrodes used, e.g. the shape and materials thereof, the size of the sample, the positional relationship between the sample and the electrode, and the kinds of substances which lie between the sample and the electrode (especially in the case of indirect stimulation). The condition settings suitable for the purpose of the present invention can be appropriately performed with, for example, making reference to the conditions described in Masuda et al. (*Gen. Pharmac*., **30**, 579-584 (1998)) by one of ordinary skill in the art.

The apparatus to be used when the contraction response is induced is not particularly limited, and it is preferable to use a Magnus apparatus. This is because there is an advantage that the enucleated organ still has intramuscular nerve plexus even when the enucleated organ is removed from the control of the central nerves, so that its mobility is not lost.

One example of the use of a Magnus apparatus will be explained.

First, with one end of a cut piece of the ciliary muscle hanged to an anchor portion located at the bottom of the Magnus tube in the tube, and the other end attached to a hook portion of the tension transducer, the Magnus tube is filled with an artificial nutrient solution prepared by a conventional method. Thereafter, a mixed gas of 5% carbon dioxide and 95% oxygen is aerated through the Magnus tube from an inlet tube. With regulating the tension with manually moving the tension transducer upwardly and downwardly, a load is previously applied to the sample, and the tube is set to the Magnus apparatus.

For instance, when acetylcholine or the like is used as a contraction agonist of the smooth muscle after a given period of time in the set Magnus tube, the agonist is added so that its first final concentration is 10⁻⁴ mol/L after 30 minutes.

Thereafter, the sample is washed at a point where the reaction reaches a plateau, and after having confirmed that the signal returns to the baseline, the stimuli by the contraction agonist are repeated.

The number of stimulations can be set so that the ciliary muscle having a desired stable state of fatiguing is obtained. Although the number is variable depending upon the quality and the intensity of the stimuli applied each time, the number of stimulations is usually preferably 3 to 50 times, more preferably 3 to 20 times, and especially preferably 4 to 15 times. A higher level of fatiguing of the ciliary muscle may be induced by increasing the repetition number, but the stimuli of more than 50 times may usually give the ciliary muscle no additional effective fatigue. In addition, usually, repetitively applying moderate stimuli in some degree is preferred to obtain a stable fatiguing.

Thirdly, the change in contraction ratio by the stimulation for the sample generated as described above is recorded.

The method of recording is not particularly limited. Concretely, the following method may be considered.

First, the tension of the sample is measured after each stimulation. Next, based on the resulting measurement values, a contraction ratio is calculated, assuming that the tension obtained by subtracting the contraction tension at the baseline from the contraction tension after the first stimulation is defined as 100%, and recorded.

Also, when the decrease in the contraction ratio of the ciliary muscle is stabilized, the ciliary muscle showing a stable decrease in the contraction ratio of the ciliary muscle to preferably 50 ± 30%, more preferably 50 ± 20%, and especially preferably 50 ± 10% is used as the sample.

When the sample in the present invention is obtained by using the Magnus apparatus with changing the chemical stimuli to the electrical stimuli or the like, the ciliary muscle showing a stable decrease in the contraction ratio of the ciliary muscle to preferably 50 ± 30%, more preferably 50 ± 20%, and especially preferably 50 ± 10%, is used in the evaluation as a sample.

Fourthly, a test formulation to be evaluated is reacted against the sample obtained as described above.

Concrete examples of the method are not particularly limited, and can be exemplified by the following method.

For instance, when the Magnus apparatus is used, the solution in the Magnus tube is replaced with an artificial nutrient solution containing a test formulation of a given concentration prior to applying a final stimulation.

Thereafter, the change in contraction ratio of the sample is recorded in the same manner as above after the treatment with the solution.

The above test may be carried out as a preliminary test using a base for the test formulation as an index prior to the main test for the test formulation. And a pharmacological comparative test for pharmacological efficacy can be carried out by comparing the results of the preliminary test with the results of the main test for the test formulation.

The test formulation is not particularly limited, and includes existing drugs of which effects have been already known or medicines which are currently newly developed of which effects have not been fully known, and the evaluation can be made on all of the medicinal effects, minimal pharmacologically effective dose and the like. As one example of this invention, there can be exemplified the formulation of vitamin B12 (cyanocobalamin) available under the trade names: Sancoba eye drop 0.02 % (manufactured by Santen Pharmaceutical Co., Ltd.), Cabalam eye drop 0.02 % (manufactured by Nippon Tenganyaku), Softear eye drop 0.02 % (manufactured by Senju Pharmaceutical Co., Ltd.), Pharcoba eye drop 0.02 % (manufactured by Toyo Pharma Kabushiki Kaisha); and the formulation of flavine adenine dinucleotide available under the trade names: FAD eye drop 0.05 % (manufactured by Santen Pharmaceutical Co., Ltd.), FAD T eye drop 0.05 % (manufactured by Nitto Meditec), Nitten FA eye drop 0.05% (manufactured by Nippon Tenganyaku Laboratories), Vitast eye drop 0,05 % and 0.1 % (manufactured by Senju Pharmaceutical Co., Ltd.), Flavitan eye drop 0.05 % (manufactured by TOA EIYO Pharmaceutical Company) and the like.

Furthermore, a comparative test on pharmacological efficacy can be carried out by conducting a test on a standard formulation as a comparative control which is used in place of the test formulation in the above main test, and comparing the results.

As the standard medicine, there can be used a medicine for ameliorating asthenopia other than that used in the above test.

The pharmacological comparative test for pharmacological efficacy can be carried out only using subjects under the same conditions. Furthermore, since the ciliary muscle of which true fatiguing is reproduced, which is induced in a living body, is used as a sample in the case of the present invention, objective and relative comparisons can be made.

### EXAMPLES

The present invention will be described hereinbelow on the bases of the following examples, and are not to be interpreted as limiting the scope of the present invention to these examples.

### (Preparation of Sample)

Eight rabbits were subjected to test as test animals after quarantine and conformation.

The rabbits were bred individually in an animal rearing box placed indoors under the breeding conditions of at a room temperature of 21° ± 3°C, relative humidity of 50 ± 20 %, using an illumination time of 12 hours (7 a.m. lights-on, 7 p.m. lights-off) and ventilation of 10-15 times per hour.

The above test animals were subjected to general anesthesia by intramuscular injection of a mixed solution of Ketalar : Seraktal (4:5) at 1 mL/kg followed by enucleation of the eyeballs.

Here, the anesthetic agents used were ketamine hydrochloride injection solution (manufactured by Sankyo Co., Ltd., specifically Ketalar 50 for intramuscular injection) and xylidine hydrochloride injection solution (manufactured by Bayer Yakuhin, Ltd., specifically Seraktal 2 % injections for dogs and cats).

Each enucleated eyeball was sclerotomized and amputated to half at the equatorial position of the eyeball, and then the ciliary muscle was carefully ablated from the sclera after removal of the crystalline lens. The ciliary muscle thus obtained was cut into a strip of 3 mm in width and 6 mm in length to give a sample.

For the above enucleation surgery, conventional ophthalmic surgical instruments such as a keratome, a micro knife, a pincette and scissors were used.

### (Generation of Fatiguing)

The sample obtained in the above process was suspended in a Magnus tube.

The Magnus tube was filled with Krebs-Henseleit solution bubbled through with a mixed gas of 95% oxygen and 5% carbon dioxide.

Krebs-Henseleit solution was prepared by mixing 1 volume of the following A-solution, 1 volume of B-solution and 1 volume of C-solution, and thereafter adding 6 volumes of distilled water thereto, with thorough mixing, and thereafter finally adding 1 volume of D-solution to the mixed solution. The resulting solution was shielded from light and heated upon use to 37°C.

The preparation methods for A- to D-solutions were as follows. A-solution: Distilled water added to 69.2 g of NaCl, 3.50 g of KCl, 1.63 g of KH₂PO₄, 2.96 g of MgSO₄•7H₂O to make up a final volume of 1000 mL. B-solution: Distilled water added to 4.00 g of glucose to make up a final volume of 200 mL. C-solution: Distilled water added to 4.20 g of NaHCO₃ to make up a final volume of 200 mL. D-solution: Distilled water added to 3.68 g of CaCl₂•2H₂O to make up a final volume of 100 mL.

The sample in the Magnus tube was first suspended at a loading weight of 0.4 g using a tension transducer (Isometric Transducer, Model FD Pick-up TB-611 T, manufactured by Nihon Kohden Corporation). After 30 minutes, acetylcholine (acetylcholine chloride, manufactured by SIGMA) was added as a first stimulation so as to give a final concentration of 10⁻⁴ mol/L. At a point where reaction reached a plateau value, the sample was washed and the reaction was confirmed to return to the baseline, and thereafter stimulation was given again in the same procedures as above. The procedures were repeated nine times.

The tension of the sample upon each stimulation was recorded by the data-collecting software (VISUAL DESIGNER Ver. 2.3, manufactured by INTELLIGENT INSTRUMENTATION) through the above tension transducer, an input box (Model JD-112S, manufactured by Nihon Kohden Corporation), an amplifier for pressure strain (Model AP-621G, manufactured by Nihon Kohden Corporation) and an A.D. conversion board (PCI-20428W, manufactured by INTELLIGENT INSTRUMENTATION).

The contraction ratio was calculated on the bases of the resulting data, assuming that the tension obtained by subtracting the contraction tension at the baseline from the contraction tension observed after the first stimulation by the contraction stimulant was 100%, and recorded.

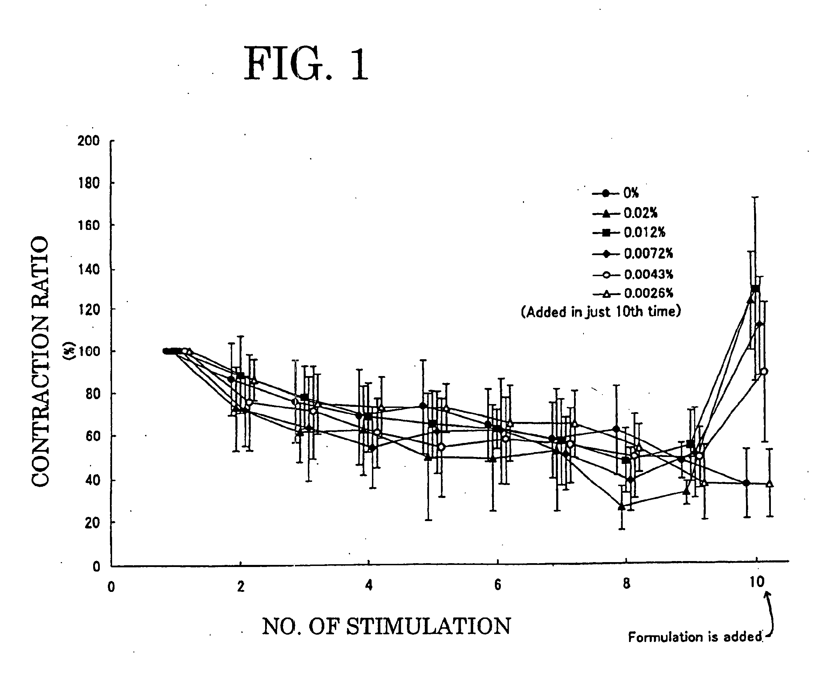

The results are shown in Tables 1-5 and in Figure 1.

In addition, for the purpose of confirming whether or not the sample was reacted to the stimulation by acetylcholine, the tension for the sample pretreated with atropine having a final concentration of 10⁻⁶ mol/L was also determined. The results are shown in Tables 6-8.

**Table 1**

| No. of measuring | Average of Contraction Ratio (%) ± standard deviation | | | | | |
|---|---|---|---|---|---|---|
| 2nd | 86.4 ± 17.3 | 72.7 ± 19.7 | 88.4 ± 18.3 | 71.5 ± 16.6 | 75.6 ± 22.4 | 86.2 ± 9.6 |
| | -- | N.S. | N.S. | N.S. | N.S. | N.S. |
| 3rd | 75.8 ± 19.5 | 61.3 ± 13.7 | 77.8 ± 14.8 | 63.3 ± 24.3 | 70.9 ± 21.7 | 74.6 ± 14.3 |
| | -- | N.S. | N.S. | N.S. | N.S. | N.S. |
| 4th | 68.5 ± 22.4 | 62.1 ± 20.7 | 68.2 ± 16.0 | 53.9 ± 18.8 | 60.8 ± 16.3 | 72.6 ± 14.5 |
| | -- | N.S. | N.S. | N.S. | N.S. | N.S. |
| 5th | 73.0 ± 21.8 | 49.6 ± 29.7 | 64.8 ± 15.6 | 61.1 ± 19.1 | 53.8 ± 23.2 | 72.2 ± 11.5 |
| | -- | N.S. | N.S. | N.S. | N.S. | N.S. |
| 6th | 64.4 ± 16.9 | 49.0 ± 24.9 | 62.5 ± 9.0 | 61.9 ± 24.4 | 57.5 ± 20.1 | 65.4 ± 17.7 |
| | -- | N.S. | N.S. | N.S. | N.S. | N.S. |
| 7th | 57.5 ± 17.4 | 52.6 ± 28.7 | 56.7 ± 20.0 | 50.9 ± 17.0 | 54.9 ± 17.2 | 64.9 ± 15.5 |
| | -- | N.S. | N.S. | N.S. | N.S. | N.S. |
| 8th | 61.9 ± 20.7 | 25.7 ± 10.3 | 47.6 ± 15-2. | 38.7 ± 14.9 | 49.6 ± 19.6 | 53.5 ± 11.0 |
| | -- | * | N.S. | N.S. | N.S. | N.S. |
| 9th | 47.5 ± 7.9 | 32.5 ± 6.0 | 54.4 ± 16.1 | 50:8 ± 21.0 | 48.9 ± 14.1 | 37.0 ± 17.5 |
| | -- | N.S. | N.S. | N.S. | N.S. | N.S. |
| Cyanocobalamin Concentration | Control 0% | 0.02% | 0.012% | 0.0072% | 0.0043% | 0.0026% |
| 10th | 36.3 ± 16.3 | 122.7 ± 23.4 | 128.3 ± 43.8 | 111.1 ± 23.7 | 88.6 ± 33.5 | 36.1 ± 16.0 |
| | -- | ** | ** | ** | * | N.S. |

| | | | | | | |
|---|---|---|---|---|---|---|
| **:P<0.01, *:P<0.05, N.S.:no significant (by Dunnett multiple comparison) | | | | | | |

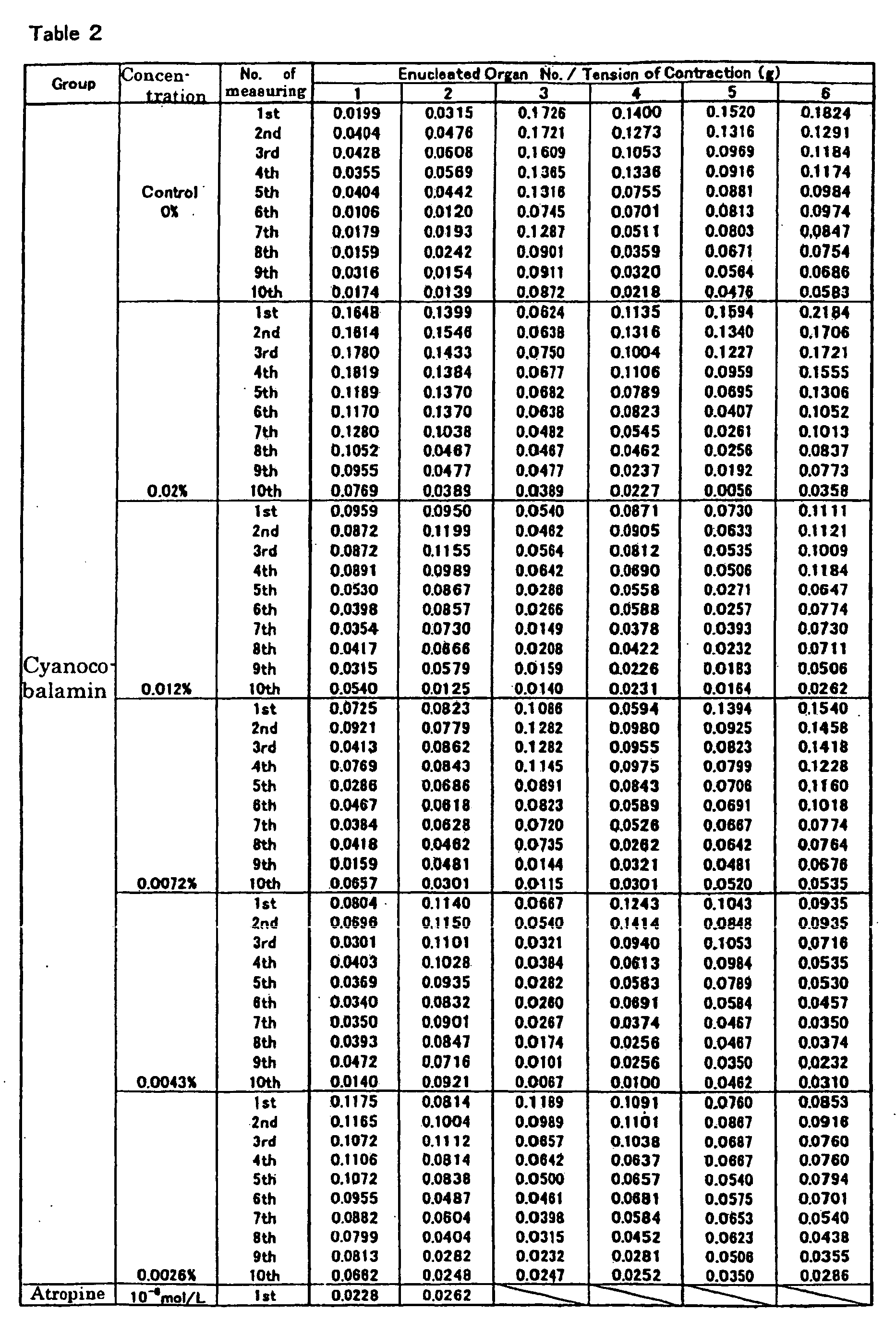

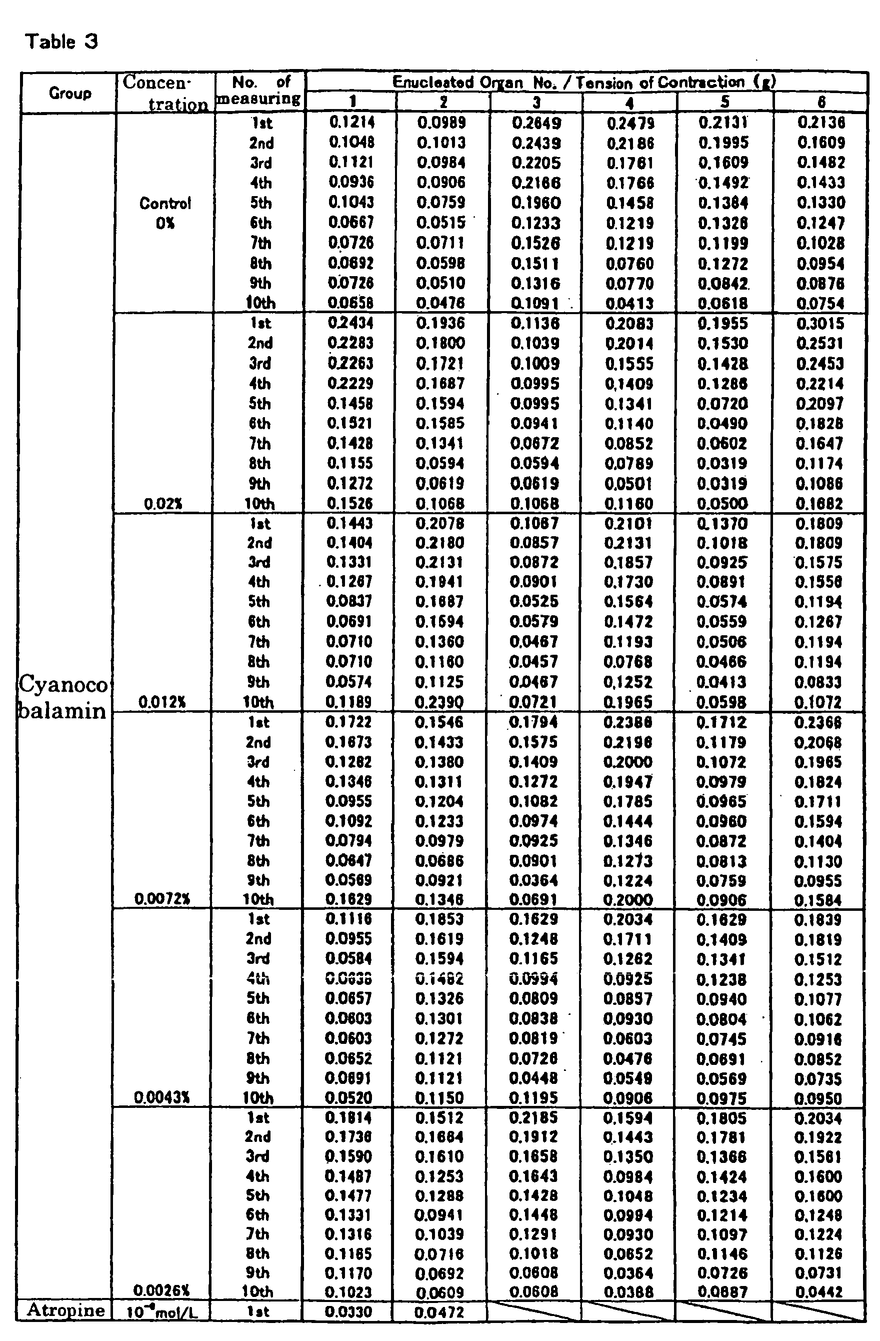

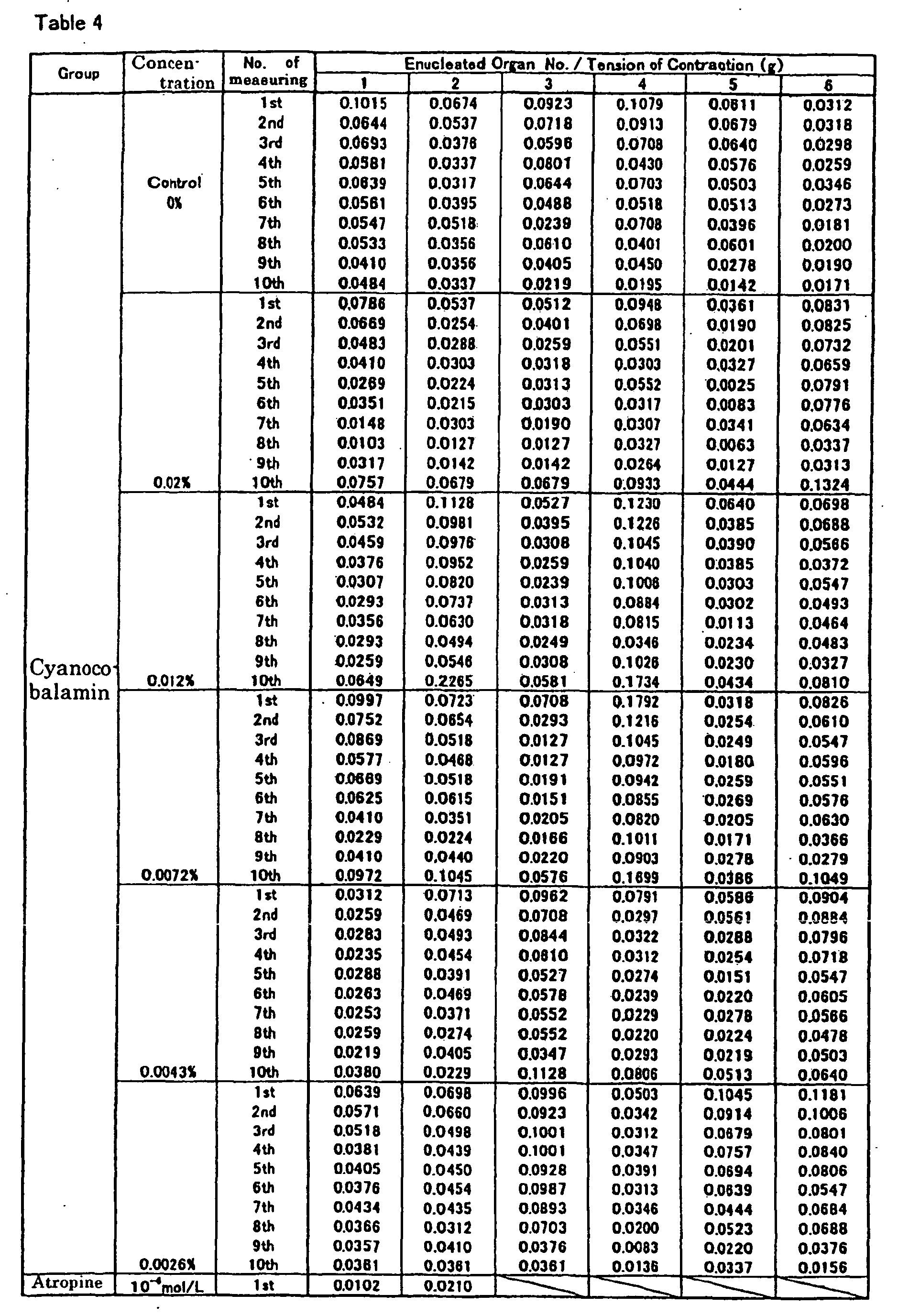

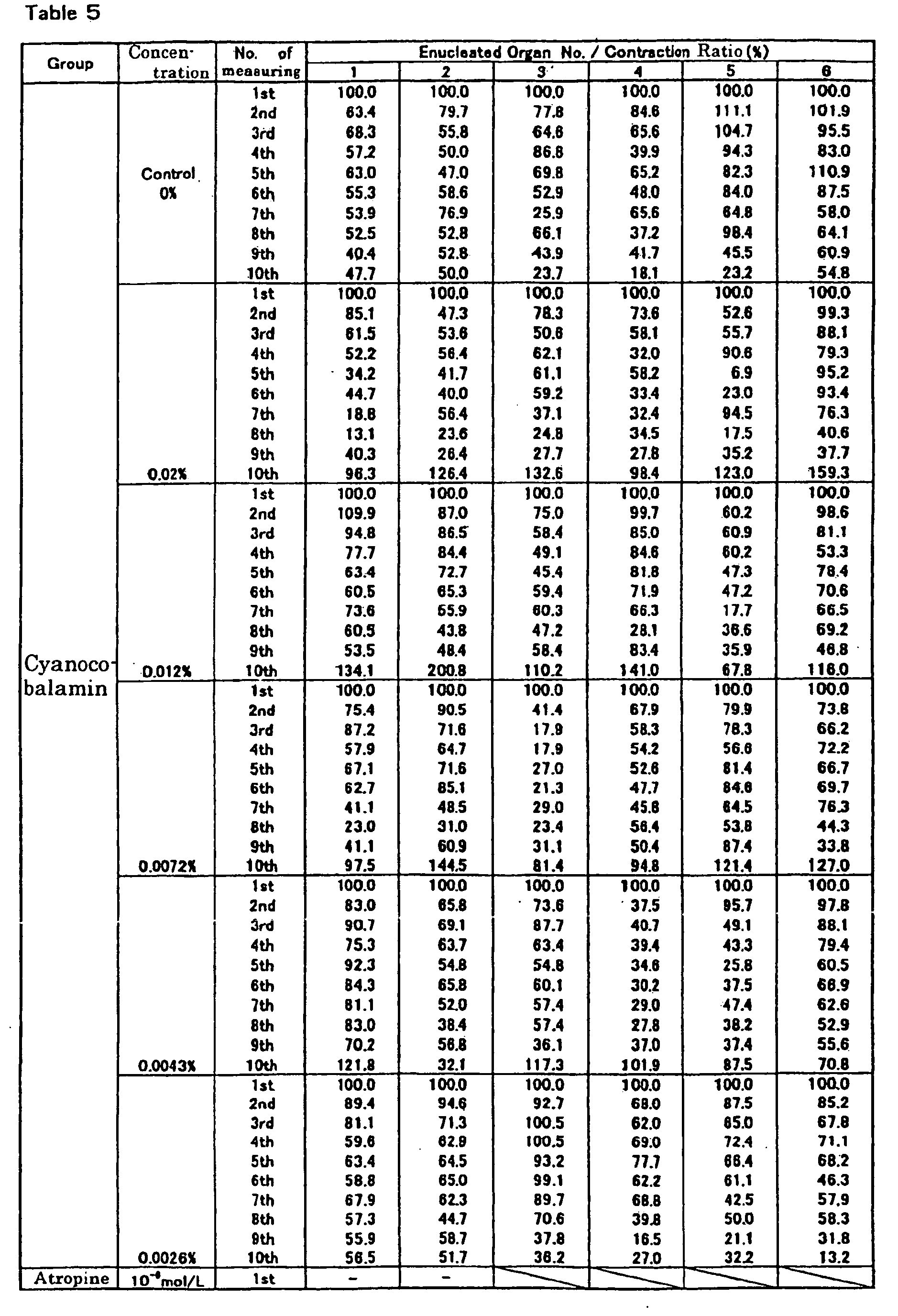

**Table 6**

| Concentration | Enucleated Organ No./Tension of Contraction (g) | | Average ± standard deviation | Inhibition Ratio (%) |
|---|---|---|---|---|
| | 1 | 2 | | |
| Control | 0.1487 | 0.1693 | 0.1590 ± 0.0146 | - |
| 10⁻⁸ mol/L | 0.0228 | 0.0262 | 0.0245 ± 0.0024 | 84.6 |

**Table 7**

| Concentration | Enucleated Organ No./Tension of Contraction (g) | | Average ± standard deviation | inhibition Ratio (%) |
|---|---|---|---|---|
| | 1 | 2 | | |
| Control | 0.1829 | 0.2630 | 0.2230 ± 0.0566 | - |
| 10⁻⁶ mol/L | 0.0330 | 0.0472 | 0.0401 ± 0.0100 | 82.0 |

**Table 8**

| Concentration | Enucleated Organ No./Tension of Contraction (g) | | Average ± standard deviation | Inhibition Ratio (%) |
|---|---|---|---|---|
| | 1 | 2 | | |
| Control | 0.0342 | 0.0937 | 0.0640 ± 0.0421 | - |
| 10⁻⁸mol/L | 0.0102 | 0.0210 | 0.0156 ± 0.0076 | 75.6 |

It is seen as shown in Tables 1-5 and in Figure 1 that fatiguing of the ciliary muscle can be generated because the contraction ratio is decreased, or the contraction tension is decreased by a repeat of stimulations by acetylcholine. Also, it can be confirmed from Tables 6-8 that the contraction reaction by acetylcholine is a reaction via muscarinic receptor because the contraction reaction of the ciliary muscle is induced by acetylcholine and inhibited by atropine pretreatment, whereby it is seen that acetylcholine is a contraction stimulant very close to that of physiological conditions.

### (Preliminary Test)

After the above stimulations, before the application of the 10th stimulation, the solution in the Magnus tube was replaced with a Krebs-Henseleit solution containing cyanocobalamin at each concentration of 0%, 0.02%, 0.012%, 0.0072%, 0.0043% and 0.0026 %, and the tension of the sample was recorded in the same manner as mentioned above.

The reason why cyanocobalamin was used in this preliminary test was that cyanocobalamin is the active ingredient of the test formulation used in the following main test.

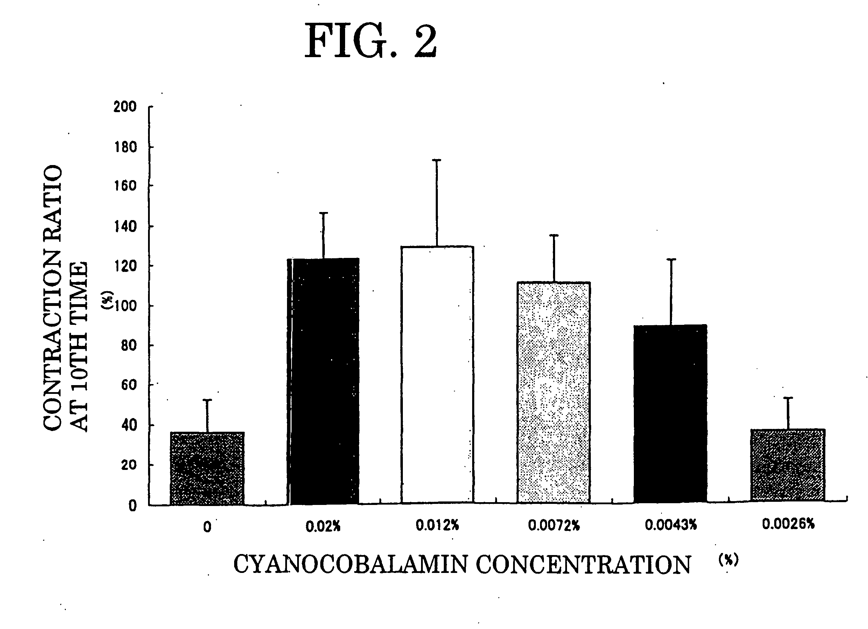

The results are shown in Tables 1-5 and Figures 1 and 2.

The Krebs-Henseleit solution containing cyanocobalamin was prepared as follows. Specifically, 1 volume of the A-solution, 1 volume of the B-solution and 1 volume of the C-solution were mixed, and thereafter 5 volumes of distilled water and 1 volume of a 10-fold concentrate of cyanocobalamin solution were added thereto with thorough mixing. Finally, 1 volume of the D-solution was added thereto. The prepared solution was shielded from light and heated upon use at 37°C.

It is seen as shown in Tables 1-5 and Figure 1 and Figure 2 that there is no influence observed with 0.0026 % cyanocobalamin, but the decrease in contraction reaction by acetylcholine is inhibited concentration-dependently from 0.0043 %. Also, it is seen as shown in Table 2 that cyanocobalamin at a concentration of 0.0043% or higher does not influence the baseline tension for any of the concentrations, showing that the cyanocobalamin itself at a concentration of 0.0043% or higher does not give direct action, and has an anti-fatiguing effect. Also, it is seen that the anti-fatiguing effect by cyanocobalamin substantially reaches a plateau at 0.012%.

For the above reasons, the concentration of the index medicine cyanocobalamin to the main test was set at 0.012% and used as an index in the following main test.

### (Main Test)

The same test as used in Preliminary Test was carried out, but using 0.02% cyanocobalamin eye drop TP263 (manufactured by Toyo Pharma Kabushiki Kaisha) as a test formulation, and Sancoba eye drop 0.02% (manufactured by Santen Pharmaceutical Co., Ltd.) as a standard formulation, in place of cyanocobalamin. The results were evaluated using the Tukey multiple comparative method.

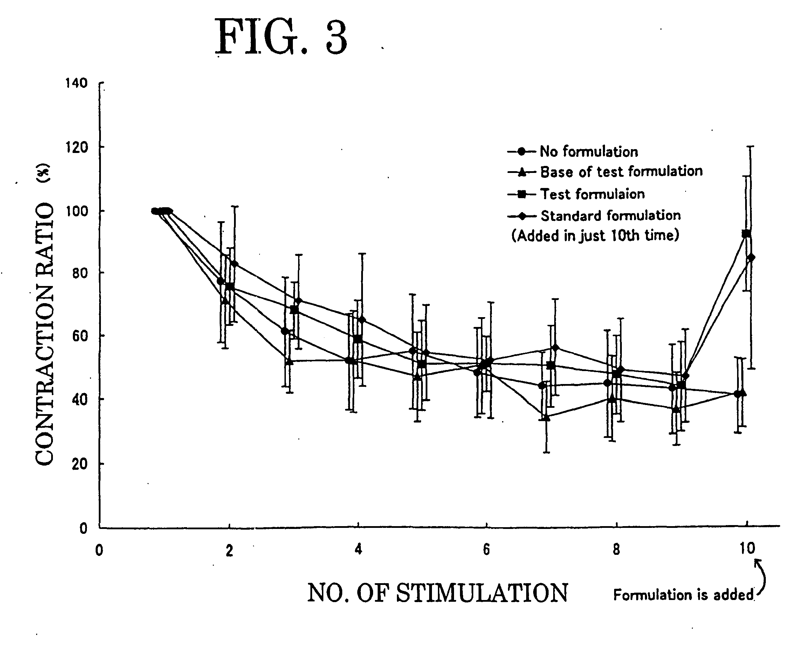

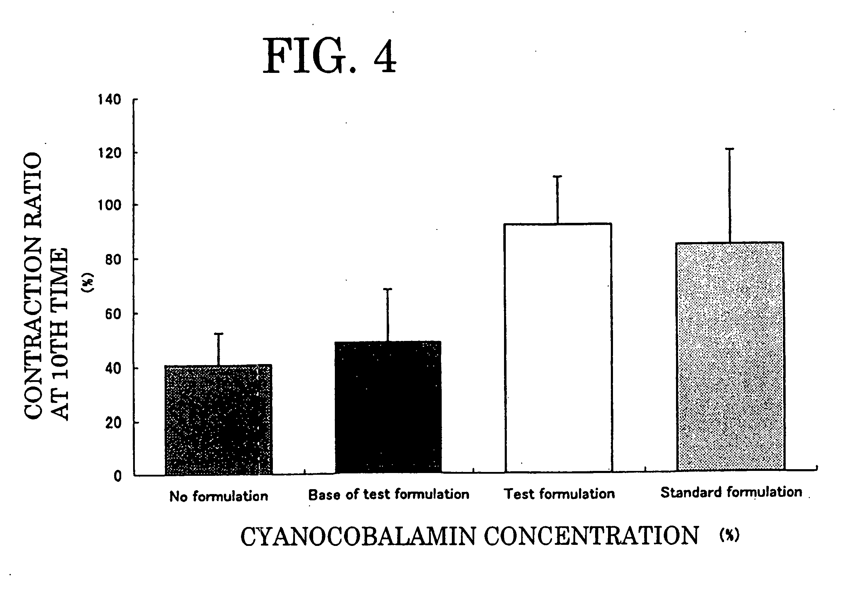

The results are shown in Table 9 and Figure 3 and Figure 4.

**Table 9**

| Existence of formulation | No. of stimulation | Average ± standard deviation | Tukey multiple comparison | | | |
|---|---|---|---|---|---|---|
| None | 2nd | 77.3 ± 19.2 | 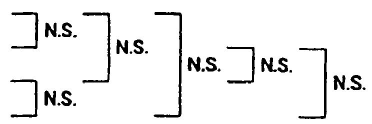 | | | |
| | | 76.3 ± 17.6 | | | | |
| | | 75.7 ± 12.3 | | | | |
| | | 83.1 ± 18.6 | | | | |
| None | 3rd | 61.2 ± 17.3 | 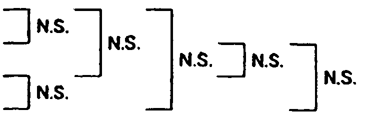 | | | |
| | | 55.1 ± 12.1 | | | | |
| | | 68.0 ± 9.0 | | | | |
| | | 70.8 ± 14.9 | | | | |
| None | 4th | 51.6 ± 15.1 | 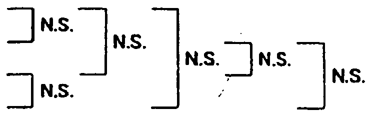 | | | |
| | | 56.6 ± 17.7 | | | | |
| | | 58.6 ± 12.2 | | | | |
| | | 64.8 ± 21.0 | | | | |
| None | 5th | 54.7 ± 18.0 | 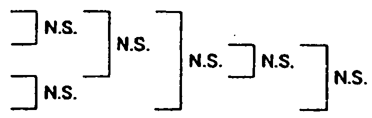 | | | |
| | | 50.4 ± 13.8 | | | | |
| | | 50.5 ± 14.1 50.5 ± 14.1 | | | | |
| | | 54.3 ± 14.9 | | | | |
| None | 6th | 48.1 ± 14.1 | 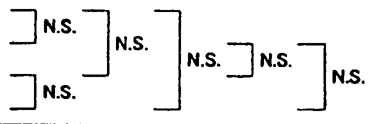 | | | |
| | | 55.4 ± 16.6 | | | | |
| | | 50.9 ± 8.8 | | | | |
| | | 52.0 ± 18.2 | | | | |
| None | 7th | 44.0 ±10.7 | 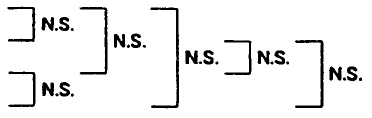 | | | |
| | | 39.6 ± 16.6 | | | | |
| | | 50.3 ± 12.8 50.3 ± 12.8 | | | | |
| | | 56.2 ± 15.2 | | | | |
| None | 8th | 44.7 ± 16.7 | 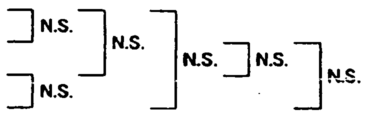 | | | |
| | | 43.8 ± 13.6 | | | | |
| | | 47.6 ± 12.3 | | | | |
| | | 49.0 ± 16.3 | | | | |
| None | 9th | 43.0 ± 14.0 | 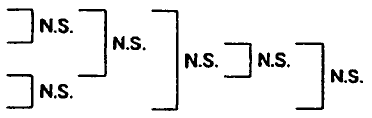 | | | |
| | | 41.7 ± 14.4 | | | | |
| | | 43.8 ± 14.0 | | | | |
| | | 46.9 ± 14.5 | | | | |
| No formulation Base of test formulation Test formulation Standard formulation | 10th | 40.7 ± 11.7 | 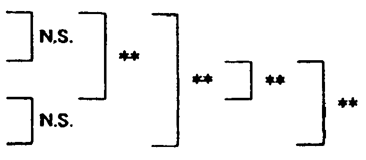 | | | |
| | | 48.4 ± 19.3 | | | | |
| | | 91.9 ± 18.2 | | | | |
| | | 84.2 ± 35.4 | | | | |

| | | | | | | |
|---|---|---|---|---|---|---|
| **:P<0.01, *:P<0.05, N.S.:no significant | | | | | | |

It was seen according to the results in Table 9 and Figure 3 and Figure 4 that the test formulation has an anti-fatiguing effect on the ciliary muscle, and that its effects are higher than that of the base for test formulation.

Furthermore, it is seen in the comparison of the test formulation with the standard formulation that both of the test formulation and the standard formulation have an anti-fatiguing effect on the ciliary muscle, and the effects are of the same level.

### INDUSTRIAL APPLICABILITY

The model of the present invention is useful as a model which can be applied to evaluation for a medicine for ameliorating asthenopia, as described in the section of "Means to Solve the Problems" in the present specification. Further, there are provided a method of generating the model of the present invention and a method for evaluating a medicine using the sample of the present invention. Providing an excellent evaluation system is indispensable in the development of an excellent medicine, which is an important invention leading to provide the excellent medicine.

The in vitro model of the present invention has excellent characteristics of (i) being capable of evaluating under conditions close to those of physiological conditions, while being an in vitro test; (ii) being capable of stably and conveniently obtaining a sample having desired fatiguing with high reproducibility by changing the conditions during the generation of the model; (iii) being capable of quantitatively determining action strength of a test substance; and (iv) being a model closer to actual asthenopia as compared to those of conventional methods.

Conventional in vitro model (for instance, a model described in Japanese Patent Laid-Open No. Hei 7-133225) is suitable for a system for carrying out evaluation or screening of a medicine showing an antagonistic action against transient ciliary muscle contraction. However, since asthenopia is not caused by transient ciliary muscle contraction, it cannot be said to be suitable as the evaluation system of an agent for ameliorating asthenopia. Also, since it is a system in which a test medicine is added before induction of ciliary muscle contraction (preventive administration system), the model is suitable as the evaluation system for medicine for preventing muscle contraction (prophylactic medicine), but cannot be said to be suitable as an evaluation system for therapeutic medicines.

On the other hand, the model of the present invention is an evaluation system using ciliary muscle in a stable fatiguing obtained by inducing contraction and relaxation in the ciliary muscle a plurality of times, which is a model using ciliary muscle in a far closer state to an actual asthenopia state as compared to those of conventional models. Also, since the model is a system in which a medicine is added after the generation of fatiguing (therapeutic administration system), it is suitable as an evaluation system for therapeutic medicines.

Furthermore, the evaluation method using the sample of the present invention has excellent characteristics of (i) being capable of carrying out evaluation of the effect of a medicine with high reproducibility; (ii) being capable of quantitatively determining the effect of a medicine; (iii) facilitating comparison of action strength of a standard medicine and a test medicine; (iv) being useful for screening not only for a known medicine but also a novel medicine.

## Claims

1. An experimental model system comprising of a non-human enucleated ciliary muscle in the state of asthenopia for evaluating the effect of a medicine against asthenopia in vitro, wherein said asthenopia is caused by contracting an enucleated ciliary muscle repeatedly by the use of smooth muscle contraction-inducing means to induce repeated contraction thereof in vitro until said ciliary muscle shows a substantially stable decrease of 50 ± 30% in the tension of muscle contraction.

2. The experimental model of claim 1, wherein said ciliary muscle shows a decrease of 50 ± 20% in the tension of muscular contraction.

3. The experimental model of claim 1, wherein said ciliary muscle shows a decrease of 50 ± 10% in the tension of muscular contraction.

4. The experimental model of claim 1, 2 or 3, wherein said smooth muscle contraction-inducing means is a chemical stimulant selected from the group consisting of acetylcholine, serotonin, histamine, muscarine, nicotine and endothelin.

5. A method for evaluating a medicine against asthenopia, **characterized by** contacting the model for asthenopia as defined in any one of claims 1 to 4 with a medicine, and measuring a contraction ratio of ciliary muscle, thereby evaluating an effect of ameliorating asthenopia by the medicine.

6. A method according to claim 5, **characterized by** measuring contraction ratios of ciliary muscle before and after administration of the medicine, and comparing the contraction ratios.

7. The method according to claim 5 or 6, **characterized by** carrying out the method by the use of Magnus apparatus.

## Patentansprüche

1. Versuchsmodellsystem, umfassend einen nicht humanen, enukleierten Ziliarmuskel im Zustand der Asthenopie, zum Bewerten der Wirkung einer Arznei gegen Asthenopie in vitro, wobei die Asthenopie durch wiederholte Kontraktion eines enukleierten Ziliarmuskels unter Verwendung eines die Kontraktion von glatten Muskeln induzierenden Mittels bewirkt wird, um dessen wiederholte Kontraktion in vitro zu induzieren, bis der Ziliarmuskel eine weitgehend stabile Abnahme von 50 ± 30 % der Muskelkontraktionsspannung zeigt.

2. Versuchsmodell nach Anspruch 1, wobei der Ziliarmuskel eine Abnahme von 50 ± 20 % der Muskelkontraktionsspannung zeigt.

3. Versuchsmodell nach Anspruch 1, wobei der Ziliarmuskel eine Abnahme von 50 ± 10 % der Muskelkontraktionsspannung zeigt.

4. Versuchsmodell nach Anspruch 1, 2 oder 3, wobei das die Kontraktion von glatten Muskeln induzierende Mittel ein chemisches Stimulans ist, das aus der Gruppe bestehend aus Acetylcholin, Serotonin, Histamin, Muskarin, Nikotin und Endothelin ausgewählt ist.

5. Verfahren zur Bewertung einer Arznei gegen Asthenopie, **gekennzeichnet durch** das Kontaktieren des Modells für Asthenopie, wie in einem der Ansprüche 1 bis 4 definiert, mit einer Arznei und das Messen eines Kontraktionsverhältnisses des Ziliarmuskels, wodurch die Wirkung einer Verschlechterung der Asthenopie **durch** die Arznei bewertet wird.

6. Verfahren nach Anspruch 5, **gekennzeichnet durch** das Messen der Kontraktionsverhältnisse des Ziliarmuskels vor und nach der Verabreichung der Arznei und das Vergleichen der Kontraktionsverhältnisse.

7. Verfahren nach Anspruch 5 oder 6, **gekennzeichnet durch** das Durchführen des Verfahrens unter Verwendung des Magnus-Apparats [Magnus apparatus].

## Revendications

1. Modèle expérimental d'un système comprenant un muscle ciliaire énucléé non humain au stade d'asthénopie pour évaluer l'effet d'un médicament contre l'asthénopie où l'asthénopie est provoquée par la contraction de manière répétitive d'un muscle ciliaire énucléé en utilisant un moyen induisant une contraction lente du muscle pour provoquer une contraction répétitive de celui-ci in vitro jusqu'à ce que ledit muscle ciliaire montre une diminution sensiblement stable de 50±30% de la tension de contraction du muscle.

2. Modèle expérimental selon la revendication 1, **caractérisé en ce que** le muscle ciliaire montre une diminution de 50±20% de la tension de contraction musculaire.

3. Modèle expérimental selon la revendication 1, **caractérisé en ce que** le muscle ciliaire montre une diminution de 50±10% de la tension de contraction musculaire.

4. Modèle expérimental selon la revendication 1, 2 ou 3, **caractérisé en ce que** ledit moyen induisant une contraction lente du muscle est un stimulant chimique choisi dans le groupe constitué par l'acétylcholine, la sérotinine, l'histamine, la muscarine, la nicotine et l'endothéline.

5. Méthode d'évaluation d'un médicament contre asthénopie, **caractérisée en ce qu'**on met en contact le modèle tel que défini dans l'une quelconque des revendications 1 à 4 avec un médicament, et on mesure le ratio de contraction dudit muscle ciliaire, pour évaluer ainsi l'effet d'amélioration d'asthénopie par le médicament.

6. Méthode selon la revendication 5, **caractérisée en ce qu'**on mesure les ratios de contraction du muscle ciliaire avant et après administration du médicament et on compare les ratios de contraction.

7. Méthode selon la revendication 5 ou 6, **caractérisée en ce qu'**on met en oeuvre la méthode en utilisant l'appareil de Magnus.
